# EUROPEAN PATENT APPLICATION

(11) **EP 2 183 967 A1**
(43) Date of publication of application: **12.05.2010**
(21) Application number: 09013977.5
(22) Date of filing: 06.11.2009
(51) Int. Cl.: A01M 1/20, A61L 9/03, A61L 9/12

(54) **Liquid transfer and evaporation device and wicks therefor**

(30) Priority: 07.11.2008 US 112409 P
(71) Applicant: Givaudan SA, 1214 Vernier (CH)
(72) Inventor: Litten-Brown, Colin, RG42 2BD Bracknell (GB); Shaukat, Anjum Fatima, Middlesex TW2 6RX (GB)
(74) Representative: Givaudan Patents

(57) **Abstract**

Improved transfer elements (wick, emanator) comprising plant material including material derived from the stems of plants of the family Fabaceae selected from a plant of the genus Aeschynomene, a plant of the genus Sesbania, and a plant of the genus Robinia, and devices having at least one such transfer element including air fresheners and evaporation devices for other actives (fragrances, insecticides, fungicides, pharmaceuticals). Methods of assembling a device having at least one transfer element, and for using the transfer element or device to transfer or evaporate liquids containing active are also provided.

## Description

Improved transfer elements comprising plant stem material from plants of the family Fabaceae (also known as Leguminosae, belonging to the order Fabales, class Magnoliopsida, and phylum Magnoliophyta), from the genus Aeschynomene, genus Sesbania and/or Robinia, and their use for the transfer and evaporation of liquids comprising actives, such as fragrances, insecticides, fungicides, and pharmaceuticals, from a reservoir to the ambience, are provided.

### BACKGROUND

In WO07098627, applicant disclosed the use of sola plant material from the plant family Fabaceae as versatile transfer elements (wick, emanator) with a good absorbency that can efficiently transfer both aqueous and non-aqueous liquids comprising actives at a good evaporation rate.

A remaining problem both with the transfer element materials disclosed in WO07098627 as well as other natural material-based transfer elements is that they grow naturally and accordingly do not have regular cross-sectional segments. A particular problem with sola material is that it is very soft and therefore difficult to precisely cut into the desired shape. However, for compatibility with reservoirs and dispensing elements of e.g. airfresheners or other dispensing devices for actives including, without limitation, pharmaceuticals and insecticides, an exact fit is highly desirable.

Another problem of sola material is that it is brittle and fragile and even when a precise cut is achieved it easily bends out of shape or snaps, especially when a long relatively thin cylindrical shape is desired, as is often the case for airfreshener transfer elements such as wicks.

Accordingly, there remains a need for a similary versatile and effective transfer element (wick, emanator) material that has a good absorbency and can efficiently transfer both aqueous and non-aqueous liquids comprising actives at a good evaporation rate, without the drawbacks of the soft, brittle fragile material.

### SUMMARY

Surprisingly it has been found that sola transfer elements can be modified so that they provide the desired stability but retain their good transfer characteristics, in particular, their good absorbency and the ability to efficiently transfer both aqueous and non-aqueous liquids, for example transferring liquids comprising actives at a good evaporation rate.

It was found that cutting and rolling of sola material (and the resulting potential compression of the material) does not impair the liquid transfer characteristics of the material (compare example 1), and therefore allow to form transfer elements that have a more regular cross section and optionally are strengthened.

Provided is the following:
(1) A transfer element that comprises dried plant stem material, wherein the transfer element comprises said dried plant stem material in form of at least one rolled-up sheet.
(2) The transfer element as described herein, including under (1), wherein the dried plant stem material is from a plant of the family Fabaceae selected from a plant of the genus Aeschynomene, a plant of the genus Sesbania, and a plant of the genus Robinia.
(3) The transfer element as described herein including under (1) and (2) which is a wick.
(4) The transfer element as described herein including under (1) and (2) which is an emanator.
(5) The transfer element as described herein including under (1) to (4) wherein the transfer element further comprises at least one support member.
(6) The transfer element as described herein including under (1) to (5) wherein the support member comprises one or more materials selected from the group consisting of plastic, metal, glass, ceramic, wood, rattan, bamboo, cellulose, textile, or combinations thereof.
(7) A device for the transfer and evaporation of a volatile liquid comprising:
   a reservoir for said volatile liquid; and
   at least one transfer elements as defined herein including under any one of (1) to (6).
(8) A device for the transfer and evaporation of a volatile liquid provided in form a combination of its components a) and b) ready for assembly upon use, comprising:
   a) a sealed reservoir filled with a volatile liquid; and
   b) at least one transfer element as defined herein including under any one of (1) to (6).
(9) The device as described herein including under any one of (7) and (8), wherein said at least one transfer element is selected from the group consisting of a wick, an emanator, and combinations thereof.
(10) The device as described herein including under any one of (7) and (8), wherein said reservoir is filled with a liquid comprising at least one active selected from the group consisting of fragrance, insecticide, fungicide, pharmaceutical, and combinations thereof.
(11) The device as described herein including under any one of (7) to (10), wherein at least one of said transfer elements is provided at least at one end with a point-like structure sufficiently pointed to be punched through the seal of the reservoir by manual pressure.
(12) The device as described herein including under (11), wherein said point-like structure is provided by modification of the form of the wick to provide said point-like structure, or by addition of a second material to provide said point-like structure.
(13) The device as described herein including under (12) wherein the at least one transfer element comprises a support member and wherein the second material to provide the point-like structure is or is part of the support member.
(14) A method of providing a device as defined herein including under any one of (7) and (9) to (13), comprising assembling at least one transfer element and the reservoir to provide the device.
(15) A method of providing a device as defined herein including under any one of (8) to (13), comprising assembling of the reservoir and at least one transfer element to provide said device upon first use.
(16) A method of disseminating a volatile liquid from a reservoir into an atmosphere comprising transferring and evaporating the liquid by means of at least one transfer element, wherein the at least one transfer element is defined as described herein under any one of (1) to (6).
(17) A method of disseminating a volatile liquid from a reservoir into an atmosphere by employing a device as defined herein under any one of (7) to (13).
(18) The method as described herein including under any one of (16) and (17), wherein said at least one transfer element is selected from the group consisting of a wick, an emanator, and combinations thereof.
(19) The method as described herein including under any one of (16) to (18), wherein said volatile liquid comprises at least one active selected from the group consisting of fragrance, insecticide, fungicide, pharmaceutical, and combinations thereof.
(20) Use of dried plant stem material from a plant of the family Fabaceae selected from a plant of the genus Aeschynomene and a plant of the genus Sesbania as a transfer element in a device for the transfer of a volatile liquid, wherein said dried plant stem material is used in form of at least one rolled-up sheet.
(21) Use as described herein including under (20) wherein the transfer element is selected from the group consisting of a wick and an emanator.

### DETAILED DESCRIPTION

A transfer element transfers a volatile liquid to be disseminated into an ambient atmosphere. Transfer elements include wicks and emanators. A wick is a transfer element that transfers a volatile liquid from a volatile liquid reservoir to a surface where the volatile liquid evaporates into an ambient atmosphere, or to an emanator. An emanator is a transfer element that transfers volatile liquids from a wick into an ambient atmosphere by evaporation.

To achieve a transfer element having a more regular form/cross-section, a length of a sola stem (typically about 5 cm to about 15 cm, for example 10 cm), preferably after at least partial removal of the outer skin, is cut into a thin strip or sheet of regular width, typically about 0.25 to about 7 mm, for example about 1 to about 5 mm. The cutting of sheets can be performed using a sharp blade, for example in form of a plane-like implement adjusted in size according to the diameters of the stems. Alternatively, any other method that provides thin sheets can be used, for example a very thin metal wire or thin filament of other material can be used. The cut most efficiently is performed parallel to the outer surface, discarding any irregular beginning of the sheet (especially if the outer skin has not already been removed and the sola stem has not been smoothed, for example by sanding). While more sola material will be lost, it is also possible to cut longitudinal sections and remove the sections towards beginning and end of the stem.

After any irregular beginning or end of the sheet is removed, a rectangular sheet for example about 10 cm wide and with a length depending on the original diameter of the stem and the thickness at which the sheet was cut remains. This sheet can then be cut to any desired length. One or more of the resulting thin sheets are then rolled into a cylindrical form. Applying minimal pressure, a rolled-up sheet without (or with only minimal) gaps where sheet surfaces touch can be achieved, for example, without limitation, rolling sheets by hand or machine. Alternatively, sheets can be rolled more loosely, eiter over their whole length or partially, for example at one end to achieve flower-like shapes. For example, more than one transfer element loosely-rolled at one of its ends can combined to achieve the impression of a composite-type flower, with the more loosely rolled ends pointing outwards.

Rolling sheets allows for accurate control of the dimensions of the transfer element. The smaller the width/thinner the sheet or sheets, the more accurately can the dimensions of the resulting transfer element be controlled. The length of the sola stem and resulting sheet is chosen depending on the desired length of the resulting transfer element. If using more than one sola sheet to form a transfer element, they can be arranged completely or partially overlapping. An overlapping arrangement could be made such that the length of the resulting transfer element exceeds that of each of the sola sheets used. For example, a number of sola stems could be inserted into each other, each forming a connection with the next due to partial overlap. The overlap should be sufficient to provide the desired stability. However, using nonoverlapping sheets will provide transfer elements with a more regular form/cross-section.

The resulting transfer elements of regular cross-section can be strengthened in while or afterwards they are formed, by introducing one or more rigid support member before, during or after the rolling process. The easiest way would be to insert the support at the beginning of the rolling process so that the support is located in the middle of the transfer element. However, a single or multiple support members can be inserted at any point in the roll.

Typical materials for the support member include, without limitation, plastic, metal, glass, ceramic, wood, rattan, bamboo, cellulose, textile or any other material that can be formed into a relatively rigid structure of regular dimensions. Composite materials are also possible. Composites can be joined using two or more materials, for example, by using adhesives/glues, or by joining them by physical connection, for example bending or weaving metal wire around wood or cardboard or similar, optionally using adhesives or other fastening means.

The shape of the support member can vary widely depending on the desired shape of the transfer element. When forming a cylindrical transfer element the support member is typically round in cross-section.

To provide a transfer element made of natural materials only, as is preferred by many consumers, natural material should be used for the support member, for example wood, rattan, bamboo or cellulose.

Both natural materials, cardboard and ceramic have the advantage to provide some wicking properties of their own, while the sola sheet part of the transfer element was found to retain its good transfer characteristics, in particular, its good absorbency and the ability to efficiently transfer both aqueous and non-aqueous liquids comprising actives at a good evaporation rate.

Plant stem material that is useful for preparing the transfer element is taken from plants of the family Fabaceae, of the genus Aeschynomene or Sesbania. These include, without limiation, a number of species often called jointvetch, shola or sola, for example Aeschynomene afraspera (sola pith), Aeschynomene americana (American joint-vetch, joint-vetch or pega pega), Aeschynomene aspera (sola pith plant, sola), Aeschynomene falcate (Australian joint-vetch), Aeschynomene indica (curly indigo, hard sola, Indian joint-vetch, kat sola, northern joint-vetch, or sensitive joint-vetch) and Aeschynomene villosa. The stem material of sola plants is very light in weight and contains a characteristic central pith.

Furthermore, useful Aeschynomene and/or Sesbania species include, for example, without limitation, the following:

### Genus Aeschynomene:

A. abyssinica, A. acapulcensis, A. acutangula, A. afraspera, A. americana, A. amorphoides, A. angolense, A. aphylla, A. aspera, A. batekensis, A. baumii, A. bella, A. benguellensis, A. bracteosa, A. bradei, A. brasiliana, A. brevifolia, A. brevipes, A. bullockii, A. burttii, A. carvalhoi, A. chimanimaniensis, A. ciliata, A. compacta, A. crassicaulis, A. cristata, A. curtisiae, A. deamii, A. debilis, A. deightonii, A. denticulata, A. dimidiata, A. egena, A. elaphroxylon, A. elegans, A. evenia, A. falcata, A. fascicularis, A. filosa, A. fluitans, A. fluminensis, A. foliolosa, A. fulgida, A. gazensis, A. genistoides, A. glabrescens, A. glauca, A. goetzei, A. gracilipes, A. gracilis, A. grandistipulata, A. guatemalensis, A. heurckeana, A. hintonii, A. histrix, A. indica, A. interrupta, A. inyangensis, A. katangensis, A. kerstingii, A. langlassei, A. latericola, A. lateritia, A. laxiflora, A. leptophylla, A. leptostachya, A. lorentziana, A. lyonnetii, A. magna, A. marginata, A. martii, A. maximistipulata, A. mediocris, A. megalophylla, A. mimosifolia, A. minutiflora, A. mollicula, A. monteiroi, A. montevidensis, A. mossambicensis, A. mossoensis, A. multicaulis, A. nana, A. neglecta, A. nematopoda, A. nicaraguensis, A. nilotica, A. nivea, A. nodulosa, A. nyassana, A. nyikensis, A. oligophylla, A. oroboides, A. palmeri, A. paludosa, A. paniculata, A. paraguayensis, A. pararubrofarinacea, A. parviflora, A. patula, A. paucifolia, A. paucifoliolata, A. petraea, A. pfundii, A. pinetorum, A. pleuronervia, A. pluriarticulata, A. podocarpa, A. pratensis, A. pringlei, A. pseudoglabrescens, A. pulchella, A. purpusii, A. pygmaea, A. racemosa, A. rehmannii, A. rhodesica, A. riedeliana, A. rivularis, A. rosei, A. rostrata, A. rubrofarinacea, A. rubroviolacea, A. rudis, A. ruspoliana, A. sansibarica, A. scabra, A. schimperi, A. schindleri, A. schliebenii, A. scoparia, A. selloi, A. semilunaris, A. sensitiva, A. siifolia, A. simulans, A. solitariiflora, A. sparsiflora, A. standleyi, A. stipitata, A. stipulosa, A. stolzii, A. tambacoundensis, A. tenuirama, A. tenuis, A. trigonocarpa, A. tsaratanensis, A. tumbezensis, A. uniflora, A. unijuga, A. upembensis, A. venulosa, A. vigil, A. villosa, A. virginica, A. viscidula, A. vogelii, A. warmingii, A. weberbaueri.

### Genus sesbania:

S. aculeate, S. aegyptica, S. benthamiana, S. bispinosa, S. brachycarpa, S. brevipedunculata, S. campylocarpa, S. cannabina, S. chippendalei, S. cinerascens, S. coerulescens, S. concolor, S. dalzielii, S. drummondii, S. dummeri, S. emerus, S. erubescens, S. exasperata, S. formosa, S. goetzei, S. grandiflora, S. greenwayi, S. hepperi, S. herbacea, S. hirtistyla, S. hobdyi, S. javanica, S. keniensis, S. leptocarpa, S. longifolia, S. macowaniana, S. macrantha, S. macrophylla, S. macroptera, S. madagascariensis, S. microphylla, S. notialis, S. oligosperma, S. pachycarpa, S. paucisemina, S. procumbens, S. punicea, S. quadrata, S. rostrata, S. roxburghii, S. sericea, S. sesban, S. simpliciuscula, S. somaliensis, S. speciosa, S. sphaerosperma, S. subalata, S. sudanica, S. tetraptera, S. tomentosa, S. transvaalensis, S. uliginosa, S. virgata, S. wildemanii.

The plant material of some plants is more easily harvested, as they produce a softer wood with a higher percentage of spongy pith. Usually the aquatic species produce such woods, some examples from the sesbania species are S. javanica , S. sericea, S. bispinosa, S. procumbens, and S. uliginosa. In contrast, non-aquatic species, for example, S. cannabina, S. concolor, S. sesban, S. aegyptica, S. grandiflora, produce harder woods with little spongy tissue.

Many Aeschynomene, Sesbania, or Robinia species are very closely related or even identical; the following Sesbania species are also known as Aeschynomene, among other synonyms:
- Sesbania cannabina (Retz.) Pers. (also known as Aeschynomene cannabina Retz., Coronilla cannabina (Retz.) Willd., Coronilla cochinchinensis Lour., Sesban aculeata xxx var. cannabina (Retz.) Baker, Sesban australis F. Muell., Sesban cannabinus (Retz.) Poir., Sesban cochinchinensis (Lour.) DC., Sesban sericea Domin, non (Willd.) Link, Sesbania aculeata (Willd.) Pers. var. cannabina (Retz.) Baker, Sesbania sericea (Willd.) Link var. glabra Domin.);
- Sesbania cannabina (Retz.) Pers. var. cannabina (also known as Aeschynomene cannabina Retz. , Sesban cannabinus (Retz.) Poir. , Sesbania sericea (Willd.) Link var. subsinguliflora Domin)
- Sesbania cannabina (Retz.) Pers. var. sericea (Benth.) N. T. Burb. (also known as Sesbania aculeata (Willd.) Pers. var. sericea Benth.).
- Sesbania sesban (L.) Merr. (also known as Aeschynomene sesban L., Emerus sesban (L.) Kuntze , Sesban aegyptiaca Poir., Sesbania aegyptiaca Poir., Sesbania confaloniana (Chiov.) Chiov., Sesbania pubescens sensu auct.);
- Sesbania sesban (L.) Merr. subsp. sesban var. sesban (also known as Aeschynomene sesban L., Sesban aegyptiacus Poir., Sesbania aegyptiaca (Poir.) Pers., Sesbania atropurpurea Taub. , Sesbania confaloniana Chiov. , Sesbania pubescens auct., non DC., Sesbania punctata auct., non DC., Sesbania tchadica Chev.);
- Sesbania bispinosa wight (also known as Aeschynomene aculeata Schreb. , Aeschynomene bispinosa Jacq., Coronilla aculeata Willd., Sesban aculeatus Poir., nom. illeg. , Sesbania aculeata Pers., nom. illeg. , Sesbania bispinosa (Jacq.) Spreng. ex Steud., Sesbania cannabina Merr.);
- Sesbania javanica Miq. (also known as Aeschynomene paludosa Roxb., Sesbania aculeata (Willd.) Pers. var. paludosa (Roxb.) Baker, Sesbania paludosa (Roxb.) Prain, Sesbania roxburghii Merr.);
- Sesbania procumbens Wigh & Arnot (also known as Aeschynomene procumbens (Roxb.));
- Sesbania sericea, Sesbania sericea (Willd.) Link var. glabra Domin see under Sesbania cannabina (Retz.) Pers.
- Sesbania sericea (Willd.) Link var. inermis Domin, see under Sesbania cannabina (Retz.) Pers. var. sericea (Benth.) N. T. Burb.
- Sesbania sericea (Willd.) Link var. subsinguliflora Domin see under Sesbania cannabina (Retz.) Pers. var. cannabina
- Sesbania grandiflora (L.) Pers. (also known as Aeschynomene coccinea L. f., Aeschynomene grandiflora (L.) L. (GRIN), Agati coccinea (L. f.) Desv., Agati grandiflora (L.) Desv. (GRIN), Coronilla coccinea (L. f.) Willd., Coronilla grandiflora (L.) Willd., Dolichos arboreus Forssk., Emerus grandiflorus (L.) Kuntze, Resupinaria grandiflora (L.) Raf., Robinia grandiflora L. (GRIN), Sesban grandiflorus Poir. (GRIN) , Sesbania grandiflora (L.) Poir.);
- Sesbania speciosa Taub. (also known as Sesbania hildebrandtii Taub. ex Engl., Sesbania pubescens DC. var. grandiflora Vatke).

Other related plants that also have a central pith, in particular those with aquatic stems with a distinct pith surrounded by spongy parenchyma, for example of the genus Robinia, including, for example, Robinia pseudoacacia L., may be similarly useful in the invention.

Throughout this application, the abovementioned plants are referenced as "sola" or "sola plants." Sola plants are found growing around the world, usually in wet regions such as rice fields and are often regarded as weeds. The stems of the sola plant, usually stripped of the outer skin, are used in a number of decorative household items and are commonly processed into decorative shapes. The stems are commercially available cropped (usually by hand) to rods of average diameter from 20 - 50 mm with variable and irregular cross-sections.

The sola stems may be used as wicks in dried, crude form, or they may be first sanded, shaved, or stripped to at least partially or completely remove the outer skin. The skinned stem material, particularly the central pith of the stems, is very light in weight, easily compressible and very porous. Alternatively, they may be shaped by any convenient means to give rods of regular cross-section. Such means include, for example, routing, sawing, planing, cutting, lathing or forming in a die. The regular cross-section may by any desired shape, such as circular, elliptical, square, rectangular or polygonal.

Thus, the plant stem material from the sola plants can be readily formed into appropriate sizes and shapes suitable for wicks. For example, sola wicks with diameters of 2 - 50 mm, for example 2 - 20mm ( +/- 0.25-1.0 mm) or 20 - 50 mm, and a length of 2 to 60 cm, for example 2 to 15, or 3 to 10 cm, may be used as a transfer element in a device for the transfer and evaporation of a volatile liquid into an atmosphere.

By these methods, rods of relatively regular and even cross-section are achieved. Such rods may be formed from crude plant stems or commercially purchased with an evened out cross-section and in the desired length and diameter.

A sola stem may be selected according to length and diameter so that it can be inserted into a reservoir, which contains a liquid to be transferred. The stem may enter the reservoir through a suitable orifice, either directly or via a liquid-tight insert fitting into the orifice. Such an insert may be of any suitable material that is not affected by the liquid, for example solvent-resistant plastics, which include, for example, polypropylene, polyethylene and the like. The insert may have a hole slightly smaller than the wick diameter and the wick is inserted with a tight fit as slight compression of the wick does not impair performance. Alternatively, the insert can be formed of a 2-piece or hinged unit that clips around the wick before insertion into the bottle.

According to certain illustrative embodiments, air fresheners and insecticide devices, liquid is transferred from a reservoir to the air by evaporation from the surface of the wick or an associated emanator with a separate diffusing surface. The wick or emanator or both comprise soft plant stem material, for example, without limitation from Fabaceae plants selected from the genus Aeschynomene and Sesbania. The porous element used to achieve the transfer from the reservoir to the emanator/air is commonly referred to as a wick.

To disseminate a volatile liquid from a reservoir into an atmosphere, a wick may itself act as the emanator for the liquid, with the liquid evaporating from the wick surface.

Alternatively, the wick may be in contact with an emanator, whereby the wick transfers the liquid from the reservoir through the wick to the emanator and evaporation is enhanced by the larger surface of the emanator. The emanating surface may be any suitable surface made of any suitable material. For example, it may be an absorbent surface in liquid transfer contact with and extending from the surface of the stem. Such absorbent surfaces may be self-supporting, for example, cardboard, or supported, for example, a fibrous material supported on a plastic surface. It may also be a capillary sheet of the type described in WO 2004/082726.

Still alternatively, the wick may be formed in a shape to provide an enlarged surface-area itself from which the liquid can evaporate, so that no separate emanator is needed.

The device may comprise multiple transfer elements as described herein, for example a wick and an emanator as described herein. The emanator may be formed in any shape. As will be apparent to the skilled person, the shape should have a high surface area to facilitate evaporation. For example, various flower-like shapes including petal-shaped chips of sola stems arranged to form a rose-shaped structure are suitable and provide a functional as well as aesthetically pleasing emanator.

Alternatively, the transfer elements described can be combined with conventional transfer elements, for example, a conventional emanator may be used with a wick as described herein, or a conventional wick, for example a wick made of porous material well known in the art may be used together with an emanator as described herein.

One example is a rod-shaped wick of rolled-up sola plant material, optionally with a support member, without an additional emanator structure that evaporates the liquid from the wick surface. Alternatively the liquid may be evaporated by an emanator, for example in the shape of a flower. Another example is a wick that is itself flower-shaped and may be floating on a "pond-type" reservoir that is wider than its height, wherein the bottom part is exposed to the liquid, and transfers the liquid through the structure to the petal-shaped evaporation surface at the top part of the structure. Each substructure/"petal" can be made up of a thin sheet of sola partially rolled up, optionally around a support member, with individual petals/mini-transfer elements forming one larger structure functioning both as wick and emanator in form of a flower shape. The sola sheets can be rolled more loosely at the end pointing towards the ambience and/or a number of individual sola sheets can be arranged to achieve a flower-type shape, for example a composite flower shape using multiple sheets arranged for example in a circle.

The emanator provides an emanating surface that comprises material of sola plant stems as described hereinabove. This emanating surface may be constructed of a number of single elements and formed in such a way as to resemble a flower head or other decorative structure. The elements may be of identical, similar or different shape. The elements can be affixed to each other with a suitable affixing means including, for example, mechanical and chemical affixing means, in any suitable way to form an emanator. For example, they may be bound or tied together (for example by suitable bindings including thread, string, wire or tape); glued together by adhesive, stapled together, or affixed using pins or other affixing means. When glue or adhesive is used, care should be taken not to impair the transfer of liquid between wick and emanator. Alternatively, a number of single elements may be inserted into a common element formed from sola material that holds the other single elements together. Still alternatively, the single elements may be inserted/plugged into each other or into a wick, optionally using additional affixing means. For example, the upper end of the wick and the lower portion of the emanator could be fitted with rigid docking pieces that fit together and, in doing so, bring the wick and emanator into intimate contact.

The lowest portion of said emanator is arranged such in the device that it is exposed to the liquid or can contact a (usually upper) portion of the wick to allow liquid transfer. Ideally, any elements of the emanator not in direct contact with the wick should be in direct contact with elements that are in contact with the wick to allow liquid transfer between elements. The affixing means should be arranged in such a way as to avoid hindering of the liquid transfer. The emanator can be placed on top of a wick extending from a reservoir of liquid. The emanator can be kept in place relying of gravity or secured to the wick and/or to the reservoir by any suitable means, for example as described hereinabove for affixing the single elements comprised in the emanator. The skilled person will be aware that when using affixing means that potentially can hinder the liquid transfer, care must be taken to place the affixing means accordingly, for example a connector may be glued to a portion, for example an outside portion, of wick and emanator so as to leave another portion, for example an inside portion, free for liquid transfer.

The emanator may be contained within an open, closed or partly closed protective support, cover or cage. The protective support, cover or cage may, for example, be formed of a mesh, grill or transparent material so that the emanator can be seen but not touched and contact of consumer to the liquid is avoided. The protective support, cover or cage can be attached by any suitable means. A sufficiently heavy cage will be held in place by gravity when placed on the reservoir. Alternatively, various affixing means may be used as will be apparent to the skilled person, for example a screw thread mechanism or clip mechanism to the liquid reservoir, or any of the affixing means described hereinabove.

Transfer elements (wicks and emanators) of sola plant stems as hereinabove described can easily be used with various types of well-known devices for the transfer and evaporation of volatile liquids. These devices typically comprise a reservoir adapted to store volatile liquid and a porous element for transfer of the volatile liquid (wick), and optionally an emanator. The wick may directly provide the volatiles to the atmosphere via an evaporation surface. Alternatively, evaporation may be assisted by additional means, for example by an additional evaporation surface in contact to the wick so that the volatile liquid is transferred to be evaporated from the additional surface (for example a sheet or screen of porous and/or capillary material as described in WO 2005/044321 and WO 01/23008, or an emanator formed from sola plant material as hereinabove described). Additionally or alternatively, an air current can be generated by a fan or heat can be applied to speed up evaporation.

The reservoir and wick may be rendered spillage-proof by employing a seal and/or by providing that the wick fits tightly into the reservoir at the place where it leaves the reservoir. For example, a sleeve surrounding at least part of the wick as described in WO03092750 and WO0123008 may be present. The sleeve fits in with the opening of the reservoir and optionally an additional sealing means to provide a tight fit and seal the reservoir opening against spillage, and prevent or lessen spillage through the wick when the device is tilted or turned. The sleeve is made of a material impervious to the volatile liquid, and it surrounds at least a part of the wick but leaves the top and bottom portion free for liquid transfer and/or evaporation.

In the case of a complete sealing of the wick within the opening of the reservoir, a pressure vent in the reservoir may be needed to equilibrate pressure when the device is in operation.

The transfer element (wick and/or emanator) may be pre-inserted into the liquid reservoir, for example within a filled reservoir that may be sealed or provided with a cap against spillage, or within a reservoir without liquid, which is adapted to receive ato a cartridge or refill comprising the liquid.

Alternatively, the transfer element (wick and/or emanator) is provided in combination with a closed (sealed or capped) reservoir with volatile liquid, with instructions that the transfer element be inserted into the reservoir by the consumer upon first use. For example, the liquid reservoir may be provided with a breakable seal through which the transfer element is inserted. To break the seal more easily, the transfer element may be provided with one end adjusted to form a point. This may be reached by providing the transfer element with a pointed end made of sola plant stem material (outer layer/bark of older stems) or another sufficiently rigid material, for example plastic, metal, glass, ceramic, wood, rattan, bamboo, cellulose, textiles or combinations thereof. When using a transfer element with support member, said support member may be adjusted into a tapered or pointed shape so that it allows to break the seal easily by applying manual pressure. A cap may be provided to cover the wick and/or emanator and stop evaporation when not in use.

There now follows a series of non-limiting examples that serve to further illustrate the transfer elements, devices and methods. The illustrative examples should not be construed to limit the devices and methods in any manner.

### Examples

### Example 1

### Comparison of solid and rolled-up transfer elements, non-aqueous system

A length of a solid sola log/stem (commercially available from SB Enterprise, Kolkata, India), in precut form with the outer skin removed, was compared to a rolled cylinders (made from 1mm thick sola sheets of material the same batch of sola logs, cut parallel to the surface spiralling inwards by a sharp blade and handrolled tightly without leaving a gap applying minimal pressure). Both wick transfer elements had a diameter of 0.5 inch and a length of 10 cm. A reservoir contained 30 ml of a test fragrance composition with 50% fragrance in 50% Dowanol DPM (non-aqueous system) (Oust Outdoor Scent, SC Johnson, Racine, Wisconsin ).

The wick transfer elements were both immersed in the fragrance with 5 cm of each wick protruding from the neck of each reservoir which was otherwise closed to the ambience. The weight loss through evaporation was measured daily, and the cumulative weight loss in g is shown in Table 1 (each performed in duplicate).

| | **Cumulative Weight Loss (g)** | | | |
|---|---|---|---|---|
| **Time (days)** | **Rolled TE** | **Rolled TE** | **Solid log TE** | **Solid log TE** |
| 0 | 0 | 0 | 0 | 0 |
| 1 | 0.45 | 0.48 | 0.47 | 0.44 |
| 2 | 1.06 | 1.08 | 1.17 | 1.17 |
| 3 | 1.66 | 1.69 | 1.87 | 1.88 |
| 4 | 2.21 | 2.27 | 2.49 | 2.54 |
| 7 | 3.66 | 3.79 | 4 | 4.2 |
| 8 | 4.3 | 4.51 | 4.61 | 4.91 |
| 9 | 4.98 | 5.18 | 5.23 | 5.67 |
| 10 | 5.51 | 5.74 | 5.71 | 6.26 |
| 11 | 6.05 | 6.34 | 6.27 | 6.79 |
| 14 | 7.53 | 7.88 | 7.63 | 8.51 |
| 15 | 7.83 | 8.2 | 7.98 | 8.88 |
| 16 | 8.19 | 8.65 | 8.33 | 8.94 |
| 17 | 8.41 | 8.87 | 8.58 | 9.55 |
| 18 | 8.67 | 9.13 | 8.87 | 9.85 |
| 21 | 9.22 | 9.71 | 9.54 | 10.53 |
| 22 | 9.46 | 9.97 | 9.82 | 10.83 |
| 25 | 10.39 | 11.03 | 10.74 | 11.87 |
| 28 | 11.28 | 12.36 | 11.56 | 12.88 |
| 30 | 11.75 | 12.83 | 12.12 | 13.47 |
| 32 | 12.1 | 13.18 | 12.5 | 13.88 |
| 37 | 12.57 | 13.64 | 13.05 | 14.48 |
| 42 | 12.84 | 13.92 | 13.36 | 14.82 |
| 45 | 13.54 | 14.62 | 14.16 | 15.66 |
| 50 | 14.01 | 15.1 | 14.68 | 16.22 |
| 53 | 14.76 | 15.85 | 15.53 | 17.04 |
| 57 | 15.31 | 16.45 | 16.1 | 17.54 |
| 60 | 16.17 | 17.27 | 16.9 | 18.18 |

No significant difference was seen between the rolled and solid sola wick transfer elements in their transfer rates.

### Example 2

### Comparison of solid and rolled-up transfer elements, aqueous system

The experiment was performed as described in example 1 except that an aqueous fragrance system with 18% fragrance, 82% deionised water with surfactants and emulsifiers (Airwick Decosphere Mountain Breeze, Reckitt Benckiser, Slough, UK) was used.

| | **Cumulative Weight Loss (g)** | | | |
|---|---|---|---|---|
| **Time (days)** | **Rolled TE** | **Rolled TE** | **Solid log TE** | **Solid log TE** |
| 0 | 0 | 0 | 0 | 0 |
| 1 | 4.84 | 4.67 | 3.69 | 3.23 |
| 2 | 9.62 | 9.11 | 8.95 | 7.78 |
| 3 | 13.59 | 12.83 | 13.48 | 11.9 |
| 4 | 16.62 | 15.75 | 16.8 | 14.84 |
| 7 | 20.94 | 20.86 | 21.5 | 20.28 |
| 8 | 21.47 | 21.5 | 21.83 | 21 |
| 9 | 22.13 | 22.12 | 22.34 | 21.64 |
| 10 | 22.48 | 22.47 | 22.68 | 22.05 |
| 11 | 22.89 | 22.89 | 23.14 | 22.5 |
| 14 | 23.64 | 23.81 | 23.92 | 23.48 |
| 15 | 23.92 | 24.07 | 15.24 | 23.78 |
| 16 | 24.12 | 24.42 | 24.46 | 24.07 |
| 17 | 24.34 | 24.6 | 24.66 | 24.3 |
| 18 | 24.55 | 24.76 | 24.83 | 24.56 |
| 21 | 24.92 | 24.91 | 24.99 | 24.87 |
| 22 | 25.00 | 24.96 | 25.02 | 24.92 |
| 25 | 24.92 | 24.88 | 24.92 | 24.83 |
| 28 | 24.96 | 24.9 | 24.94 | 24.84 |
| 30 | 25.06 | 25.01 | 25.04 | 24.96 |
| 32 | 25.06 | 25.02 | 25.04 | 24.96 |
| 37 | 25.04 | 24.99 | 25.03 | 24.96 |
| 42 | 25.01 | 24.97 | 25.01 | 24.95 |
| 45 | 25.09 | 25.04 | 25.08 | 25.03 |
| 50 | 25.1 | 25.05 | 25.09 | 25.04 |
| 53 | 25.12 | 25.09 | 25.12 | 25.09 |
| 57 | 25.05 | 25.00 | 25.06 | 25.01 |
| 60 | 25.07 | 25.05 | 25.07 | 25.05 |

No significant difference was seen between the rolled and solid sola wick transfer elements in their transfer rates.

### Example 3

### Comparison of solid and rolled-up transfer elements, aqueous system

The experiment was performed as described in example 1 except that a water/oil based fragrance system (23% fragrance/ 44% Dowanol DPM/33% deionised water) was used.

| | **Cumulative Weight Loss (g)** | | | |
|---|---|---|---|---|
| **Time (days)** | **Rolled TE** | **Rolled TE** | **Solid log TE** | **Solid log TE** |
| 0 | 0 | 0 | 0 | 0 |
| 1 | 1.36 | 1.34 | 1.64 | 1.58 |
| 2 | 2.38 | 2.39 | 3.06 | 2.9 |
| 3 | 3.36 | 3.39 | 4.37 | 4.12 |
| 4 | 4.24 | 4.3 | 5.54 | 5.24 |
| 7 | 6.74 | 6.79 | 8.64 | 8.26 |
| 8 | 7.95 | 8.12 | 9.9 | 9.72 |
| 9 | 9.53 | 9.72 | 11.46 | 11.38 |
| 10 | 10.81 | 11.06 | 12.73 | 12.74 |
| 11 | 12.47 | 12.69 | 14.87 | 13.7 |
| 14 | 16.54 | 16.44 | 18.25 | 17.28 |
| 15 | 17.16 | 17.15 | 18.92 | 17.96 |
| 16 | 18.02 | 18.06 | 19.64 | 18.73 |
| 17 | 18.47 | 18.52 | 20.11 | 19.22 |
| 18 | 18.93 | 18.97 | 20.57 | 19.7 |
| 21 | 19.92 | 19.96 | 21.55 | 20.79 |
| 22 | 20.36 | 20.38 | 20.97 | 21.25 |
| 25 | 22.02 | 22.44 | 23.42 | 22.58 |
| 28 | 23.51 | 23.76 | 24.49 | 23.76 |
| 30 | 24.18 | 24.36 | 25.12 | 24.51 |
| 32 | 24.61 | 24.75 | 25.44 | 24.96 |
| 37 | 25.15 | 25.21 | 25.8 | 25.48 |
| 42 | 25.41 | 25.43 | 25.94 | 25.71 |
| 45 | 25.97 | 25.9 | 26.26 | 26.15 |
| 50 | 26.21 | 26.1 | 26.39 | 26.33 |
| 53 | 27.21 | 26.35 | 26.57 | 26.54 |
| 57 | 26.59 | 26.47 | 26.62 | 26.6 |
| 60 | 26.76 | 26.65 | 26.73 | 26.72 |

No significant difference was seen between the rolled and solid sola wick transfer elements in their transfer rates.

While the transfer elements, devices incorporating the transfer elements, and methods have been described above in connection with certain illustrative embodiments, it is to be understood that other similar embodiments may be used or modifications and additions may be made to the described embodiments for performing the same function. Further, all embodiments disclosed are not necessarily in the alternative, as various embodiments may be combined to provide the desired characteristics. Variations can be made by one having ordinary skill in the art without departing from the spirit and scope of the disclosure. Therefore, the transfer elements, devices and methods should not be limited to any single embodiment, but rather construed in breadth and scope in accordance with the recitation of the attached claims.

## Claims

1. A transfer element that comprises dried plant stem material, wherein the transfer element comprises said dried plant stem material in form of at least one rolled-up sheet.

2. The transfer element of claim 1 wherein the dried plant stem material is from a plant of the family Fabaceae selected from a plant of the genus Aeschynomene, a plant of the genus Sesbania, and a plant of the genus Robinia.

3. The transfer element of any one of claims 1 and 2 which is selected from the group consisting of a wick and an emanator.

4. The transfer element of any one of claims 1 to 3 wherein the transfer element further comprises at least one support member.

5. The transfer element of any one of claims 1 to 4 wherein the support member comprises one or more materials selected from the group consisting of plastic, metal, glass, ceramic, wood, rattan, bamboo, cellulose, textile and combinations thereof.

6. A device for the transfer and evaporation of a volatile liquid comprising:
a reservoir for said volatile liquid; and
at least one transfer element as defined in any one of claims 1 to 5.

7. A device for the transfer and evaporation of a volatile liquid provided in a form of a combination of its components a) and b) ready for assembly upon use, comprising:
a) a sealed reservoir filled with a volatile liquid; and
b) at least one transfer element as defined in any one of claims 1 to 5.

8. The device of any one of claims 7 and 8, wherein said at least one transfer element is selected from the group consisting of a wick, an emanator, and combinations thereof.

9. The device of any one of claims 6 to 8, wherein at least one said transfer element is provided with a point-like structure at least at one end sufficiently pointed to be punched through the seal of the reservoir by manual pressure.

10. The device of claim 9, wherein said point-like structure is provided by modification of the form of the wick to provide said point-like structure, or by addition of a second material to provide said point-like structure.

11. The device of claim 10 wherein the at least one transfer element comprises a support member and wherein the second material to provide the point-like structure is or is part of the support member.

12. A method of disseminating a volatile liquid from a reservoir into an atmosphere comprising transferring and evaporating the liquid by means of at least one transfer element, wherein the at least one transfer element is defined as in any one of claims 1 to 5.

13. Use of dried plant stem material from a plant of the family Fabaceae selected from a plant of the genus Aeschynomene and a plant of the genus Sesbania as a transfer element in a device for the transfer of a volatile liquid, wherein said dried plant stem material is used in a form of at least one rolled-up sheet.

14. Use according to claim 13 wherein the transfer element is selected from the group consisting of a wick and an emanator.
